# NEW EUROPEAN PATENT SPECIFICATION

(11) **EP 0 674 898 B2**
(45) Date of publication and mention of the opposition decision: **22.03.2006**
(45) Mention of the grant of the patent: 23.07.2003
(21) Application number: 95200757.3
(22) Date of filing: 27.03.1995
(51) Int. Cl.: A61K 8/39, A61K 8/891, A61Q 19/10

(54) **Shampoo composition**
Shampoozusammensetzung
Composition de shampooing

(30) Priority: 31.03.1994 GB 9406555
(43) Date of publication of application: 04.10.1995
(73) Proprietor: UNILEVER PLC, London EC4P 4BQ (GB); UNILEVER N.V., 3013 AL Rotterdam (NL)
(72) Inventor: Birtwistle, David Howard, Bangkok 10110 (TH); Mavropoulou, Maria, F-95270 Asnieres sur Oise (FR)
(74) Representative: Mulder, Cornelis Willem Reinier

(56) References cited:
- EP-A- 0 400 976
- EP-A- 0 468 721
- EP-A- 0 529 883
- WO-A-95/09599
- GB-A- 2 246 575

## Description

This invention relates to shampoo compositions, particularly to hair shampoo compositions which include a silicone microemulsion and a deposition polymer.

The use of silicones in hair shampoos is well known. Generally, dispersed droplets of the silicone oil are suspended in the composition, which is then applied to the hair to deposit the silicone material on the hair shaft.

Hitherto, steps have had to be taken to prevent the emulsified droplets of silicone oil from agglomerating and the composition creaming during storage. Such steps have for example included the addition of polymers such as Carbopol or certain gums, and/or crystalline materials, e.g. ethylene glycol distearate, to act as suspending agents, but the use of such materials renders the resulting compositions cloudy or opaque. Visually and aesthetically such products are inferior.

The presence of such suspending agents in hair treatment compositions, however, is also disadvantageous because they lead to dulling of the hair, as well as lowering of other conditioning attributes, as a result of the suspending agent being deposited on the hair in addition to the intended silicone conditioning oil.

It is known in the art that oily cosmetic agents such as silicones can be incorporated into cosmetic compositions by means of microemulsification, whereby the silicone is present as stably emulsified droplets of a particle size of the order of 0.15 micrometres or less.

For example, US 4733677 discloses leave-on hair fixatives containing cationic organic polymer and polydiorganosiloxane microemulsion. EP-A-268982 describes dimethylpolysiloxane microemulsions for various cosmetic uses, the microemulsified dimethylpolysiloxane being formed by emulsion polymerization and with a particle size of 0.15 microns or less.

However, by the very nature of the form in which microemulsified particles of a conditioning oil are incorporated into cosmetic compositions, the conditioning benefits attainable are frequently limited, owing to a poor level of deposition on the intended site, ie. the hair or the skin.

In our EP A 0529883 there is disclosed a hair shampoo comprising a silicone microemulsion In combination with a cationic deposition polymer. The viscosity of the silicone microemulsion used is 15 000 centistokes. This shampoo gives satisfactory deposition of the microemulsion onto hair, but the conditioning benefit is not sufficient for many people.

In US 2 826 551, it is stated that whilst the viscosity of the polyorganosiloxane employed is not very critical, higher viscosity fluids are more effective in preventing snarling of the hair. This patent does not mention use of a cationic deposition polymer and is not concerned with the problem of getting good deposition and good conditioning from a silicone microemulsion shampoo system.

It has now been found that shampoo compositions which have good mechanical stability, high optical transparency or transluency, and excellent conditioning ability can be obtained by utilising a high viscosity microemulsified silicone oil in combination with a cationic deposition polymer.

According to the present invention there is provided an optically clear shampoo composition comprising:
(a) from 2-35% of sodium lauryl ether sulphate 3EO
(b) 0.01-10% of a microemulsion of particles of a viscosity silicone having a viscosity in excess of 50 000 mm² sec⁻¹ and a particle size of <0.15 micrometres the emulsion comprising water, emulsifier and the particles;
(c) 0.01-10% of a cationic deposition polymer which is a cationic cellulose ether devirative.

The viscosity being measured is the viscosity of the silicone itself and not that of the emulsion or the final shampoo composition. The viscosity is measured in the conventional manner using a rotary viscometer

Preferred silicones for use in the present invention include non-volatile silicones, siloxane gums and resins, aminofunctional silicones, quaternary silicones, and mixtures thereof with each other and with volatile silicones. Examples of suitable silicone polymers for use in the present invention include those disclosed in EP-A-228575.

Various methods of making microemulsions of particles of silicones for use in the invention are available and are well known and documented in the art.

One particularly preferred technique for making silicone microemulsions is that described in EP-A-228575 referred to above.

In that document there is described a method of making a stable microemulsion of high molecular weight silicone polymer and water by sequentially adding at an effective rate a standard emulsion comprising polydiorganosiloxane precursor, surfactant and waterto a polymerization catalyst medium while mixing to form a clear, stable aqueous microemulsion of polydiorganosiloxane.

Another method of making suitable microemulsions for use in the invention are described in EP-A-0 138 192.

The silicone may, for example, be a liquid at ambient temperatures, so as to be of a suitable viscosity to enable the material itself to be readily emulsified with the required particle size of 0.15 micrometres or less. However, high viscosity or even solid materials may be appropriate for use in the invention, and indeed may be preferred where in-situ polymerisation is used to prepare the microemulsified particles, as mentioned above. Alternatively, such high viscosity or solid materials may be suitable for use directly if dissolved in a water immiscible solvent. For example, in the case of a silicone which is a highly viscous silicone resin or siloxane gum, a suitable solvent is a volatile silicone or a volatile hydrocarbon. Examples of all these materials are well known to the person skilled in the art.

The amount of silicone incorporated into the compositions of the invention depends on the type of composition and the material used. A preferred amount is from 0.01 to about 10% by weight although these limits are not absolute. The lower limit is determined by the minimum level to achieve conditioning and the upper limit by the maximum level to avoid making the hair and/or skin unacceptably greasy.

The microemulsion of the silicone is stabillsed by sodium lauryl ether sulphate 3EO. Additional emulsifier, preferably chosen from anionic, cationic, nonionic, amphoteric and zwitterionic surfactants, and mixtures thereof may be present. The amount of emulsifier will typically be in the ratio of 1:1 to 1:7 parts by weight of the silicone, although larger amounts of emulsifier can be used, eg. 5:1 parts by weight of the silicone or more.

Suitable anionic surfactants are the alkyl sulphates, alkyl ether sulphates, alkaryl sulphonates, alkyl succinates, alkyl sulphosuccinates, acyl taurates, acyl glutamates, N-alkoyl sarcosinates, alkyl phosphates, alkyl ether phosphates, alkyl ether carboxylates, and alpha-olefin sulphonates, especially their sodium, potassium, magnesium, ammonium and mono-, di- and triethanolamine salts. The alkyl and acyl groups generally contain from 8 to 18 carbon atoms and may be unsaturated. The alkyl ether sulphates, alkyl ether phosphates and alkyl ether carboxylates may contain from one to 10 ethylene oxide or propylene oxide units per molecule, and preferably contain 2 to 3 ethylene oxide units per molecule.

Examples of suitable anionic surfactants include sodium oleyl succinate, ammonium lauryl sulphosuccinate, ammonium lauryl sulphate, sodium dodecylbenzene sulphonate, triethanolamine and sodium salts of dodecylbenzene sulphonate and sodium N-lauryl sarcosinate, sodium lauryl ether sulphate 1EO, 2EO, ammonium lauryl sulphate, ammonium lauryl ether sulphate 1EO, 2EO and 3EO, and triethanolamine and sodium salts of dodecylbenzene sulphonate. Sodium lauryl ether sulphate 3EO is used as it gives a particularly clear and stable shampoo when used with high viscosity microemulsions.

Suitable cationic surfactants may include quaternary ammonium hydroxides, e.g. teramethylammonium hydroxide, octyltrimethylammonium hydroxide, dodecyltrimethylammonium hydroxide, hexadecyltrimethyl-ammonium hydroxide, ocryldimethylbenzylammonium hydroxide, decyldimethylbenxylammonium hydroxide, didodecyldimethylammonium hydroxide, dioctadecyl dimethylammonium hydroxide, tallow trimethylammonium hydroxide, cocotrimethylammonium hydroxide, and the corresponding salts thereof.

Suitable nonionic surfactants may include condensation products of aliphatic (C₈-C₁₈) primary or secondary linear or branched chain alcohols or phenols with alkylene oxides, usually ethylene oxide and generally having from 6 to 30 ethylene oxide groups.

Other suitable nonionics include alkylpolyglycosides and mono- or di-alkyl alkanolamides. Examples of the latter nonionics include coco mono- or di-ethanolamide and coco mono-isopropanolamide.

Suitable amphoteric and zwitterionic surfactants may include alkyl amine oxides, alkyl betaines, alkyl amidopropyl betaines, alkyl sulphobetaines (sultaines), alkyl glycinates, alkyl carboxyglycinates, alkyl amphopropionates, alkylamphoglycinates, alkyl amidopropyl and hydroxysultaines, wherein the alkyl and acyl groups gave 8 to 19 varbon atoms. Examples include lauryl amine oxide, cocodimethyl sulphopropyl betaine and preferably lauryl betaine, cocamidoproyl betaine and sodium cocamphopripionate.

A preferred cosmetic composition in accordance with the invention is a shampoo composition which, in addition to the silicone microemulsion comprises further surfactant to provide a deterging benefit. The composition preferably comprises from about 2 to about 35% by weight in total of surfactant. The deterging surfactant is selected from anionic, cationic, nonionic, and amphoteric and zwitterionic surfactants, and mixtures thereof, examples of which are given above. The deterging surfactant may be the same surfactant as the emulsifier.

In accordance with the invention, the cosmetic composition contains a cationic cellulose ether derivative addition. Suitable addition deposition polymers are any which enhance deposition of the conditioning oil on the intended site, e.g., the hair or the scalp. Since the composition is to be an optically clear mild shampoo the deposition polymer is a cationic cellulose ether derivative because this gives good clarity and adequate flocculation on dilution with water during use, provided sufficient electrolyte is added to the formulation. Suitable electrolytes include sodium chloride and sodium benzoate.

Suitable cationic cellulose ether derivatives are quaternary ammonium derivatives of cellulose ethers, for example the Polymer JR series of materials available from Union Carbide or the Celquat materials,such as Celquat SC 240C from National Starch. Both materials have the CTFA designation POLYQUATERNIUM 10. The deposition polymer is present in an amount of from about 0.01 to about 10% by weight of the total composition, preferably from about 0.01 to about 1% by weight, even more preferably from about 0.04 to about 0.5% by weight.

The cosmetic compositions of the invention are preferably aqueous based, water forming the basis of the continuous phase of the microemulsion. The compositions preferably comprise water in an amount of from about 20 to about 99% by weight of the total composition.

The compositions of the invention are preferably rinse-off compositions, i.e., suitable for applying to the hair, left thereon for an appropriate period of time and then rinsed off with water.

Compositions in accordance with the present invention are optically clear. Depending upon the type of shampoo or silicone employed, one or more additional ingredients conventionally incorporated into shampoo formulations may be included in the compositions of the invention. Such additional ingredients include antibacterial agents, antidandruff agents, foam boosters, perfumes, colouring agents, preservatives, viscosity modifiers, proteins, polymers, buffering or pH adjusting agents, moisturising agents, herb or other plant extracts and other natural ingredients.

The invention is further illustrated by way of the following non-limiting examples.

### EXAMPLE 1

A clear hair shampoo composition (A) in accordance with the present invention, comprising a high viscosity silicone microemulsion and a cationic deposition polymer, was prepared as described herein. A similar clear hairshampoo composition (B) using a low viscosity silicone microemulsion was also prepared. The two compositions has the following formulations:

| (%wt) | | | |
|---|---|---|---|
| Ingredient | | A | B |
| | SLES 3EO (40% active) | 11.4(8) | 11.4(8) |
| | Cocoamidopropyl betaine(25% active)11(3.3) | | 11 (3.3) |
| | Silicone (1) | 3.0(0.75) | - |
| | Silicone (2) | - | 3.0(0.75) |
| | Polyquaternium 10 | 0.3 | 0.3 |
| | Salt | 1.6 | 1.6 |
| | Sodium benzoate | 0.5 | 0.5 |
| | Perfume | 0.5 | 0.5 |
| | minors | 0.33 | 0.33 |
| | Water | to 100 | to 100 |

| | | | |
|---|---|---|---|
| (1) DC 1870 dimethicone, viscosity 60 000 mm²sec⁻¹ (60 000 centistokes) added as 25% microemulsion ex Dow Corning. | | | |
| (2) DC 1865 dimethicone, viscosity 20 000 mm² sec⁻¹ (20 000 centistokes) added as 25% microemulsion ex Dow Corning. | | | |

Both compositions A and B were adjusted to a viscosity of approximately 2500-cps with salt.

Both the composition according to the invention (A) and the composition according to the prior art (B) were used to wash and condition hair which was then assessed by a number of trained panellists. Statements were put to each panellist and they gave a score of from 1 to 5 depending on how much they agreed with the statement. A score of 5 meant that they totally agreed with the statement and a score of 1 meant that they totally disagreed with the statement. The scores were then averaged and the results are given below:

| Statement | A | B |
|---|---|---|
| It leaves my hair manageable | 3.94 | 3.70 |
| It leaves my hair freshly conditioned | 4.02 | 3.77 |
| It leaves my hair feeling soft | 4.19 | 3.99 |
| It leaves build up on my hair | 2.13 | 2.28 |
| It makes my hair easy to comb when wet | 4.18 | 4.07 |
| It makes my hair easy to comb when dry | 4.04 | 3.70 |

It can be seen that the composition according to the invention gave higher score than the formulation with the low viscosity microemulsion for the attributes of: manageability, fresh conditioned, soft feel, wet comb and dry comb. It gave a lower score for the undesirable attribute of build-up.

A composition with only 1.6% of the high viscosity microemulsion (Composition C) gave similarly improved performance. Scoring even higher for manageability and conditioning and even lower for amount of build-up.

### Example 2

All three compositions used in example 1 were then tested in a comparative performance trial against a conventional silicone 2in 1 conditioning shampoo sold under the name "Sunsilk 2 in 1" and containing a mechanical emulsion of large particle size silicone with high viscosity. The prior art microemulsion composition (B) gave inferior dry combing, whereas the composition according to the invention with low level of high viscosity microemulsion gave better performance in this regard and the composition A gave even better performance. Composition A also gave better control of fly away hair than any of the other compositions.

## Claims

1. An optically clear shampoo composition comprising by weight:
(a) from 2-35% of sodium lauryl einer sulphate 3EO;
(b) from 0.01 to 10% of a microemulsion of particles of a sillcone having a viscosity in excess of 50,000 mm²sec⁻¹ and a particle size of <0.15 micrometers, the emulsion comprising water, emulsifier and the particice and
(c) 0.01 - 10% of a cationic deposition polymer which is a cationic cellulose ether derivative.

2. A composition according to claim 1, wherein the silicone is selected from non-volatile silicones, siloxane gums and resins, aminofunctional silicones, quaternary silicones, and mixtures thereof with one another and with volatile silicones.

3. A composition according to claim 1 or claim 2, wherein the particles of silicone oil have a particle size of <0.1 micrometers.

4. A composition according to any one of claims 1 to 3, wherein the microemulsified silicone oil is present in the composition in an amount of from 0.3 to 5% by weight.

5. A composition according to any preceding claim in which the emulsifier in component (b) is sodium lauryl ether sulphate 3EO.

6. A method of conditioning hair and/or skin comprising applying thereto a composition according to any preceding claim.

7. Use as an additive in an optically clear conditioning composition of a microemulsion of a silicone having a viscosity in excess of 50,000 mm²sec⁻¹ and a particle size of <0.15 micrometers In combination with a cationic deposition polymer which is a cationic cellulase ether derivative and sodium lauryl ether sulphate 3EO.

## Patentansprüche

1. Optisch klare Shampoozusammensetzung, die bezogen auf Gewicht enthält:
(a) 2-35% Natriumlaurylethersulfat-3EO;
(b) 0,01 bis 10% einer Mikroemulsion mit Silikonteilchen mit einer Viskosität von mehr als 50.000 mm²s⁻¹ und einer Teilchengröße <0,15 µm, wobei die Emulsion Wasser, Emulgator und die Teilchen enthält und
(c) 0,01 bis 10% eines kationischen Abscheidungspolymers, das ein kationisches Celluloseetherderivat ist.

2. Zusammensetzung nach Anspruch 1, wobei das Silikon ausgewählt ist aus nichtflüchtigen Silikonen, Siloxangummen und Harzen, aminofunktionellen Silikonen, quaternären Silikonen und Mischungen davon miteinander und mit flüchtigen Silikonen.

3. Zusammensetzung nach Anspruch 1 oder Anspruch 2, wobei die Teilchen aus Silikonöl eine Teilchengröße von <0,1 µm haben.

4. Zusammensetzung nach einem der Ansprüche 1 bis 3, wobei das mikroemulgierte Silikonöl in der Zusammensetzung in einer Menge von 0,3 bis 5 Gew.-% vorhanden ist.

5. Zusammensetzung nach einem der vorhergehenden Ansprüche, wobei der Emulgator in Komponente (b) Natriumlauryl-ethersulfat-3EO ist.

6. Verfahren zum Konditionieren von Haar und/oder Haut umfassend, dass eine Zusammensetzung nach einem der vorhergehenden Ansprüche darauf aufgebracht wird.

7. Verwendung einer Mikroemulsion eines Silikons mit einer Viskosität von mehr als 50.000 mm²s⁻¹ und einer Teilchengröße von <0,15 µm in Kombination mit einem kationischen Abscheidungspolymer, das ein kationisches Celluloseetherderivat ist, und Natriumlaurylsulfat-3EO als Additiv in einer optisch klaren konditionierenden Zusammensetzung.

## Revendications

1. Composition de shampoing optiquement claire comprenant, en poids :
(a) de 2 à 35 % de lauryle éther sulfate de sodium 3 EO ;
(b) de 0,01 à 10 % d'une microémulsion de particules de silicone ayant une viscosité supérieure à 50.000 mm² sec⁻¹ et une taille de particule de < 0,15 micromètres, l'émulsion comprenant de l'eau, de l'émulsifiant et les particules ; et
(c) de 0,01 à 10 % d'un polymère de dépôt cationique qui est un dérivé éther de cellulose cationique.

2. Composition selon la revendication 1, dans laquelle la silicone est sélectionnée à partir des silicones non volatiles, des gommes et des résines de siloxane, des silicones amino-fonctionnelles, des silicones quaternaires et des mélanges de celles-ci l'une avec l'autre et avec des silicones volatiles.

3. Composition selon la revendication 1 ou la revendication 2, dans laquelle les particules d'huile de silicone ont une taille de particule de <0,1 micromètres.

4. Composition selon l'une quelconque des revendications 1 à 3, dans laquelle l'huile de silicone micro émulsionnée est présente dans la composition dans une quantité allant de 0,3 à 5 % en poids.

5. Composition selon l'une quelconque des revendications précédentes, dans laquelle l'émulsifiant dans le composant (b) est du lauryle éther sulfate de sodium 3 EO.

6. Procédé de conditionnement des cheveux et/ou de la peau comprenant d'appliquer dessus une composition selon l'une quelconque des revendications précédentes.

7. Utilisation en tant qu'un additif dans une composition de conditionnement optiquement claire d'une microémulsion d'une silicone ayant une viscosité supérieure à 50.000 mm² sec⁻¹ et une taille de particules de < 0,15 micromètres, en combinaison avec un polymère de dépôt cationique qui est un dérivé éther de cellulose cationique et de lauryle éther sulfate de sodium 3 EO.
